# EUROPEAN PATENT APPLICATION

(11) **EP 3 705 492 A1**
(43) Date of publication of application: **09.09.2020**
(21) Application number: 18872706.9
(22) Date of filing: 29.10.2018
(51) Int. Cl.: C07K 14/535, C07K 19/00

(54) **METHOD FOR PREPARING, IN HIGH YIELD, CONJUGATE IN WHICH COLONY STIMULATING FACTOR AND POLYOL ARE CONJUGATED**

(30) Priority: 30.10.2017 KR 20170142363
(71) Applicant: Hankook Korus Pharmaceutical Co. Ltd., Chuncheon-si, Gangwon-do 24398 (KR)
(72) Inventor: WHANG, Jae Gan, Seoul 07703 (KR); HAN, Sang In, Incheon 21982 (KR); KWON, Sung Hun, Incheon 21120 (KR); KANG, Min Ji, Seoul 07282 (KR); KIM, Hyeong Seok, Yongin-si Gyeonggi-do 16936 (KR); JUNG, Bong Jun, Seoul 07294 (KR); HAN, Jae Hyeok, Gongju-si Chungcheongnam-do 32580 (KR); WI, Sae Mi, Seoul 06990 (KR); SEO, Kyoung Wan, Suwon-si Gyeonggi-do 16417 (KR)
(74) Representative: Ström & Gulliksson AB
(86) International application number: PCT/KR2018/012888
(87) International publication number: WO 2019/088608

(57) **Abstract**

The present invention relates to a method for preparing, in a high yield, a conjugate in which a colony stimulating factor and polyol are conjugated.

## Description

### [Technical Field]

The present invention relates to a method for preparing, in a high yield, a conjugate in which a colony stimulating factor and polyol are conjugated.

### [Background Art]

Polypeptides such as interferon alpha are generally low in stability, and thus are easily denatured and degraded by proteolytic enzymes in the blood to be easily removed by the kidneys or liver. Therefore, it is necessary to administer protein drug to the patient more frequently in order to maintain a blood concentration and titer of the protein drug including polypeptide as a pharmacological component. However, in the case of the protein drugs, which are mostly administered to patients in the form of injections, frequent injections to maintain blood concentration of the active polypeptide cause pain to a patient. To address this problem, covalent conjugation of protein and peptide to poly(ethylene glycol) (PEG) has been used. The covalent conjugation has been widely recognized as an approach to significantly extend circulatory half-life in vivo of therapeutic proteins. PEGylation primarily achieves this effect by delaying renal clearance. This is because the PEG moiety adds a significant hydrodynamic area to the corresponding protein (Zalipsky, S., et al., Use of functionalized poly(ethylene glycol)s for modification of polypeptides., in poly(ethylene glycol) chemistry: Biotechnical and biomedical applications., J. M. Harris, Ed., Plenum Press: New York., 347-370 (1992)). Additional benefits often given by PEGylating protein and peptide include increased solubility, resistance to proteolytic degradation, and decreased immunogenicity of therapeutic polypeptide.

On the other hand, in a process of preparing such a drug, in order to increase the preparing yield of the conjugate while the activity of the protein therapeutic agent is maintained, sophisticated manufacturing techniques are required. Accordingly, continuous research has been conducted on a method of preparing a protein therapeutic agent including polyethylene glycol and on polyethylene glycol used in the preparing.

The present inventors intend to provide a preparing method capable of more effectively shortening time through the present application in preparing a bioactive polypeptide conjugate using polyethylene glycol.

### [Summary of Invention]

### [Technical Problem]

An object of the present invention is to provide a method for preparing, in a high yield, a conjugate in which a colony stimulating factor and polyol are conjugated.

### [Solution to Problem]

Hereinafter, various embodiments described in the present application are described with reference to the drawings. In the following description, for the full understanding of the present invention, various specific details such as specific forms, compositions, and processes have been described. However, certain embodiments may be practiced without one or more of these specific details or in combination with other known methods and forms. In other examples, well-known processes and preparing techniques are not described in specific details in order not to unnecessarily obscure the present invention. Reference throughout the present specification to "one embodiment" or "an embodiment" means that a particular feature, a form, a composition or a characteristic described in connection with the embodiment is included in one or more embodiments of the present invention. Therefore, the context of "in one embodiment" or "an embodiment" expressed in various places throughout the present specification does not necessarily represent the same embodiment of the present invention. Additionally, special features, shapes, compositions, or properties can be combined in any suitable methods in one or more embodiments.

Unless otherwise defined in the specification, all scientific and technical terms used in the present specification have the same meaning as commonly understood by a person skilled in the art to which the present invention pertains.

In one embodiment of the present invention, "neutropenia" refers to a condition in which the level of white blood cells in the blood is reduced to below normal level (1,000 to 10,000 per 1 µl of blood). In other words, there are granulocytes such as neutrophils, eosinophil, and basophils in a white blood cell, and neutropenia means that the number of neutrophils of which the occupation ratio in the leukocyte is 50% to 70% is abnormally reduced, that is, the case where the number of neutrophils is reduced to about 1,000 or less. In other words, leukocyte includes granulocytes such as neutrophils, eosinophil, and basophils, lymphocytes, and the like, and leukopenia is usually referred to as neutropenia since the proportion occupied by the neutrophils in the leukocyte is large. In addition, an anticancer treatment such as a cancer drug and radiation lowers a bone marrow function and as a result, lowers neutrophil production to cause neutropenia in patients.

In one embodiment of the present invention, with respect to the "neutropenia treatment agent", patients with low levels of neutrophils after administration of an anticancer drug cope with infection with an antibiotic or an antifungal agent and are concurrently treated with a granulocyte macrophage-colony stimulating factor (GM-CSF) or a granulocyte-colony stimulating factor (G-CSF), which is a drug that stimulates granulocyte production and enhances the number of neutrophils. In particular, a method for preventing or treating febrile neutropenia by using a G-CSF preparation (filgrastim, lenograstim, and pegfilgrastim) is common.

In one embodiment of the present invention, "GCSF (granulocyte colony stimulating factor)" refers to a substance that stimulates the production and activation of neutrophils as a granulocyte promoter. In particular, GCSF is administered to increase the recovery of granulocytes and reduce the risk of infection after the anticancer treatment and is usually administered by intravenous injection or percutaneous injection.

In one embodiment of the present invention, "G-CSF API (granulocyte-colony stimulating factor active pharmaceutical ingredient)" is one of the granulocyte colony stimulating factors, means single chain polypeptide protein consisting of 174 amino acids, and is used for neutropenia. An active pharmaceutical ingredient means a pharmaceutically active ingredient.

In one embodiment of the present invention, "Neulasta" is commercially available from Amgen Inc. and a preparing method thereof is disclosed in Korean Patent No. 0248111. As described in the above document, in the pegylation reaction thereof, pH is adjusted to 4.0 by stirring G-CSF and mPEG in a molecular ratio of G-CSF:mPEG (1:1.5) in a 100 mM Bicine buffer at pH 8.0. With respect to the purification, in the ion exchange chromatography (Pharmacia S Sepharose FF XK 50/30 column, 440 ml volumes) equilibrated with a buffer solution A (20 mM sodium acetate, pH 4.0), protein was eluted by using 15 column volumes of a buffer B (20 mM sodium acetate, pH 4.0, 1 M NaCl) in a linear gradient from 0% to 23%. The column was then eluted by distilling the column at 1.5 ml/min for 100 min in size exclusion chromatography (Pharmacia Superdex 75 HR 16/60 column, 120 ml volumes) equilibrated with 20 mM sodium acetate at pH 4.0.

In one embodiment of the present invention, the "half-life extension" is a function that helps any compound to achieve a hydrodynamic size sufficient for preventing clearance by renal filtration in vivo. For example, a compound can be selected from a group including polymeric macromolecules in a substantially straight, branched, or dendritic form so that "half-life extension" can be obtained. On the other hand, as a compound that can achieve "half-life extension", any compound can be selected so that the compound binds to serum protein in vivo to form a complex, and thus the complex formed can avoid substantial kidney clearance. Examples of the compound that can achieve "half-life extension" include lipids, a cholesterol group (for example, steroid), carbohydrate, oligosaccharides, any natural or synthetic protein that binds to a recycling receptor, polypeptide, or peptide.

Examples of the compound that can achieve "half-life extension" that can be used in accordance with the purposes of the present invention include immunoglobulin Fc domains or parts thereof, or biologically compatible polymers or copolymers, for example, a polyalkylene glycol compound such as polyethylene glycol or polypropylene glycol. Other suitable polyalkylene glycol compounds include, but are not limited to, dextran, polylysine, colominic acid or other carbohydrate-based polymers, a polymer of amino acid, and a charged polymer or a neutral polymer such as biotin derivatives.

In one embodiment of the invention "pegylation" refers to a technique for binding a PEG derivative (biopolymer) to protein. Pegylation is a technology for increasing the stability of biopharmaceuticals by binding PEG which is a polymer backbone of a water-soluble polymer to protein and increases efficacy of the corresponding drug by increasing the half-life. The term PEG includes alkoxy PEG, difunctional PEG, multiarmed PEG, forked PEG, branched PEG, pendant PEG (that is, PEG with one or more functional groups hanging from a polymer backbone or a related polymer), or any form of poly(ethylene glycol) including PEG with degradable linkages therein. The polymer backbone can be linear or branched. Branched polymer backbones are generally known in the art. Typically, the branched polymer has a central branch core moiety and a plurality of linear polymer chains bound to the central branch core moiety. PEG is commonly used in a branched form which can be prepared by the addition of ethylene oxide to various polyols such as glycerol, pentaerythritol, and sorbitol. The central branch moiety can be derived from several amino acids such as lysine. Branched poly(ethylene glycol) can be represented as a general form as R(-PEG-OH)m, R in the above represents a core moiety such as glycerol or pentaerythritol, and m represents the number of arms.

Briefly, PEG groups are attached to a part of peptide of the compound of the present invention generally by reductive alkylation or acylation with a reactive group (for example, aldehyde, amide, or ester groups) on the compound of the present invention by a reactive group on a PEG moiety (for example, aldehyde, amide, thiol, or ester groups). Strategies useful for PEGylating synthetic peptides may be performed by combining peptide and a PEG moiety that each possess special functional groups that are mutually reactive with each other by conjugate chain formation in a solution.

Useful PEGs according to the present invention include substantially linear, straight chain PEG, side chain PEG, or dendritic PEG [see: for example, Merrill, US Patent No. 5,171,264; Harris et al., Multiarmed, monofunctional, polymer for coupling to molecules and surfaces, US Patent No. 5,932,462; Shen, N-maleimidyl polymer derivatives, and US Patent No. 6,602,498].

The present invention provides a method for preparing a conjugate in which a colony stimulating factor and polyol are conjugated, the method comprising: a step of mixing polyol and a colony stimulating factor to produce a mixture; a step of washing the mixture at pH 3.0 to 7.0 to obtain a washed object; and a step of eluting the washed object at pH 4.1 to 6.5 to obtain an eluate.

According to an embodiment of the present invention, the polyol is poly(ethylene glycol) (PEG). According to another embodiment of the present invention, the colony stimulating factor is a granulocyte colony stimulating factor (G-CSF). According to still another embodiment of the present invention, a step of mixing the colony stimulating factor in a ratio with mPEG (monomethoxy-polyethylene glycol) which is one of a precursor of the polyol to be 1:3 to prepare the mixture is further included. According to still another embodiment of the present invention, a step of using 20 mM ammonium acetate-acetic acid as a buffer in the washing is further included. According to still another embodiment of the present invention, the buffer in the washing is pH 4.8 to 6.8. According to still another embodiment of the present invention, the buffer in the washing is pH 5.8. According to still another embodiment of the present invention, a step of using 20 mM ammonium acetate-acetic acid as the buffer in the elution is further included. According to still another embodiment of the present invention, the buffer in the elution is pH 4.5 to 5.5. According to still another embodiment of the present invention, the buffer is pH 5.0 in the elution.

### [Advantageous Effects of Invention]

The present invention provides a method for preparing, in a high yield, a conjugate in which a colony stimulating factor and polyol are conjugated. According to the present invention, a newly discovered range of pH is applied to allow purification by a single-step purification process, and as a result, it was also found that an effect of purification which is equal to or higher than that of Neulasta with multiple steps is exhibited.

### [Brief Description of Drawings]

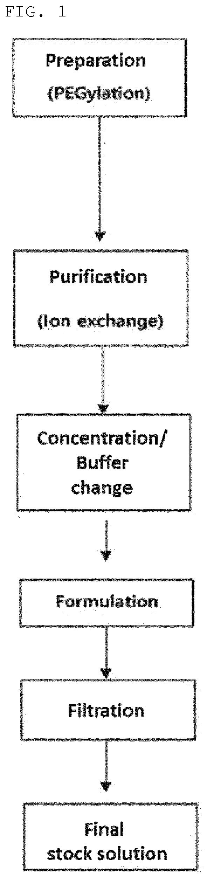

FIG. 1 is a schematic diagram of a method of preparing PEG-GCSF at a high yield according to the present invention.
FIG. 2 shows the results of electrophoresis by varying a loading volume of each by a titrisol staining method.
FIG. 3 shows the results of electrophoresis in a reduced state and a non-reduced state by a silver staining method.
FIGS. 4 shows results of analysis and confirmation by RP-HPLC by comparing Neulasta (FIG. 4(a)) with a case of the preparing method according to the present invention (FIG. 4(b)).
FIGS. 5 shows results of analysis and confirmation by SEC-HPLC by comparing Neulasta (FIG. 5(a)) with a case of the preparing method according to the present invention (FIG. 5(b)).
FIGS. 6 shows results obtained by analyzing and confirming peptide-mapping by comparing Neulasta (FIG. 6(a)) with a case of the preparing method according to the present invention (FIG. 6(b)).
FIG. 7 shows a comparison of Neulasta with the cases produced according to the preparing method according to the present invention by a western blot method.

### [Description of Embodiments]

Hereinafter, the present invention is specifically described with reference to examples. These examples are only intended to specifically describe the present invention, and it is apparent to those skilled in the art that the scope of the present invention is not limited by these examples according to the gist of the present invention.

### Example: Method for increasing a yield of PEG-GCSF 1) Arrangements

HKP (Hankook Korus Pharm) batches (#1, #2, #3, and #4) in different conditions were prepared. In order to obtain sufficiently reproducible experimental results, it was confirmed whether valid results can be obtained with repeated experiments by the same method to the batches #1 to #4. In addition, Neulasta (supplier: Amgen Inc.) was used as a positive control, and G-CSF API was used as a negative control.

### 2) Preparation

The molecular ratio of G-CSF was prepared in a ratio with mPEG (monomethoxy-polyethylene glycol) of 1:3. Next, NaCNBH₃ was added at a multiple of 10 times the molar amount of mPEG. Preferably, NaCNBH₃ was added at 10 times, 100 times, 1,000 times, and 10,000 times with respect to the molar amount of mPEG. The final concentration was determined by using a 20 mM sodium acetate-acetic acid (pH 5.0) buffer. The concentration of the substance was prepared to be 4.0 mg/ml. The prepared reagent was stirred at 4°C for 18 hours.

### 3) Purification

A reagent prepared by equilibration by 4 CV with a 20 mM sodium acetate-acetic acid (pH 5.0) buffer was purified by loading SP Sepharose resin with a flow rate of 1 cm/min for cation exchange. As the washing buffer, 20 mM ammonium acetate-acetic acid (pH 5.8) was used. When the reagent reaches pH 5.8, an elution buffer of 20 mM ammonium acetate-acetic acid (pH 5.0) was used.

### 4) Concentration

The buffer was changed to a 10 mM sodium acetate-acetic acid (pH 4.0) buffer by maximizing the concentration to 10 mg/ml.

### 5) Formulation

5% Sorbitol (final concentration is 5%) and 0.004% Tween 20 (final concentration is 0.004%) were added.

### 6) Filtration

The filter pore size was 0.2 µm, and filtration was performed by using a hydrophilic cellulose acetate filter.

### 7) Final stock solution

The final stock solution was stored at 4°C.

### Result

As described above, in the purification process of the preparing method, a target material was purified by a purification process of two or more steps in the related art. However, in the present invention, a specific pH range in which a target material can be purified by only one step is revealed to provide a preparing method with reduced time requirement and increased efficiency and improve the technical process in the related art.

Specifically, as described above, Neulasta was purified by a process of two steps of ion exchange chromatography and size exclusion chromatography by using pH 8.0 and 4.0 buffers. However, HKP produced by the method described herein was stirred at a molar ratio of G-CSF:mPEG (1:3) in 20 mM sodium acetate of pH 5.0 to perform pegylation reaction. Then, purification was performed by equilibration with a buffer A (20 mM sodium acetate, pH 5.0). Next, in the ion exchange chromatography (Pharmacia S Sepharose FF XK 26/20 column, 53 ml volumes), washing was performed with a buffer B (20 mM ammonium acetate, pH 5.8), and protein was eluted with a buffer C (20 mM ammonium acetate, pH 5.0) at pH 5.8.

In general, it is considered to be the best method if protein with high purity can be obtained in minimal purification steps in a protein purification step. According to the present invention, the loss rate of about 10% to 50% was generated as a result of only a single step of the purification step. As described above, while protein was obtained by a process of two or more steps of chromatography in the purification method of Neulasta, a condition where pH of the buffer is adjusted and only a single step of chromatography is performed was conceived in the method described in the present invention. In terms of the effect, it is considered that a yield would be better by the method of the present invention in which purification is performed by chromatography with a single step as a purity equal to or greater than that of Neulasta in the related art is exhibited.

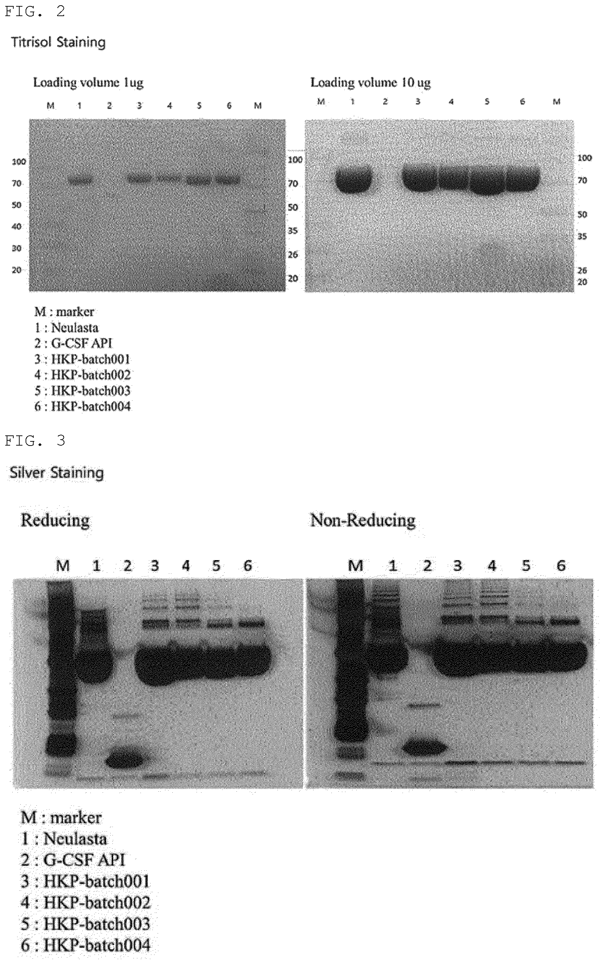

More specifically, FIG. 2 is photographs of titrisol staining that enables the confirmation of the presence or absence of PEGylated proteins of Neulasta and G-CSF API used as the control groups and an HKP sample used as a test group. The photograph shows that the band expression was exhibited at the same position in the pegylated Neulasta and HKP samples, while nothing was detected in the non-pegylated G-CSF API.

FIG. 3 is silver staining photographs that can confirm the entire molecule of Neulasta and G-CSF API used as the control group and the HKP sample used as the test group. The photograph shows that the band expression was exhibited at the same position in the Neulasta and HKP samples, while the band expression was checked at the bottom in the non-pegylated G-CSF API.

Therefore, as shown in FIGS. 2 and 3, it was observed that the test groups (HKP-batches #1, #2, #3, and # 4) obtained by the preparing method according to the present application had the same size and amount as Neulasta. This is a result indicating that, though the purification process was reduced, protein can be obtained in the same or better yield than a material in the related art.

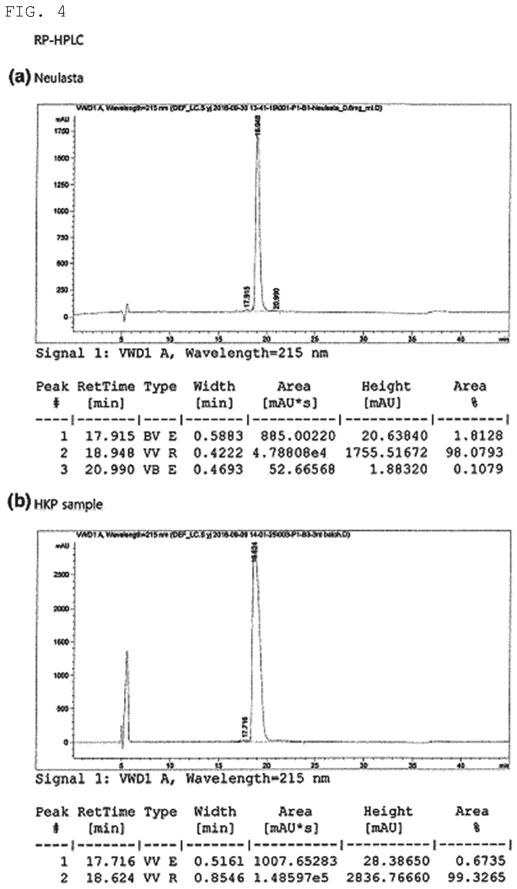

In addition, FIGS. 4 is graphs presenting results of reverse phase chromatography (RP-HPLC) exhibiting a difference in interaction according to a polarity difference of materials. It was confirmed that Neulasta as the control group and the HKP sample as the test group were detected at the same time. The purity was 98.07% and 99.32%, and thus it was confirmed that the purity of the HKP sample as the test group was higher.

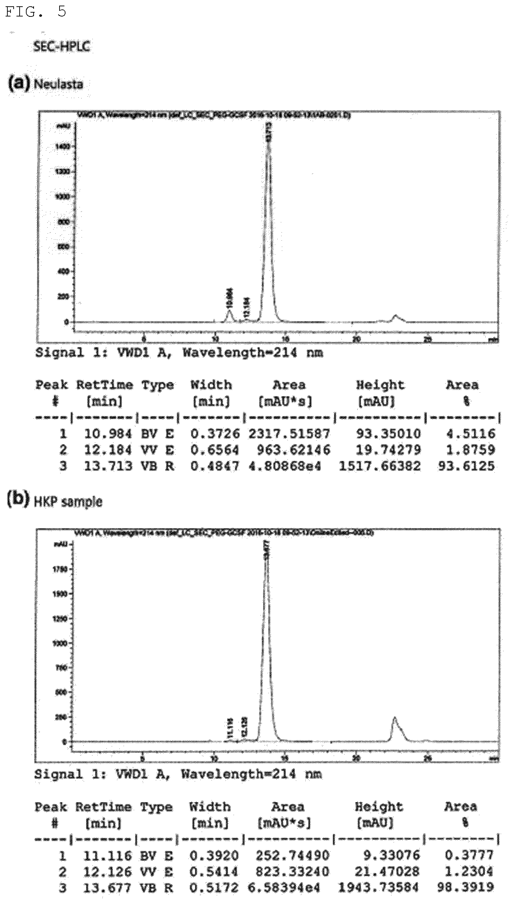

FIGS. 5 is graphs presenting results of size exclusion chromatography (SEC-HPLC) exhibiting a difference according to molecular sizes. It was confirmed that Neulasta as the control group and the HKP sample as the test group were detected at the same time. The purity was 93.61% and 98.39%, and thus it was confirmed that the purity of the HKP sample as the test group was higher.

As presented in FIGS. 4 and 5, it was confirmed that the test groups (HKP-batches #1, #2, #3, and # 4) obtained by the preparing method according to the present application were the same as Neulasta as shown by chromatography, and the purity thereof was higher.

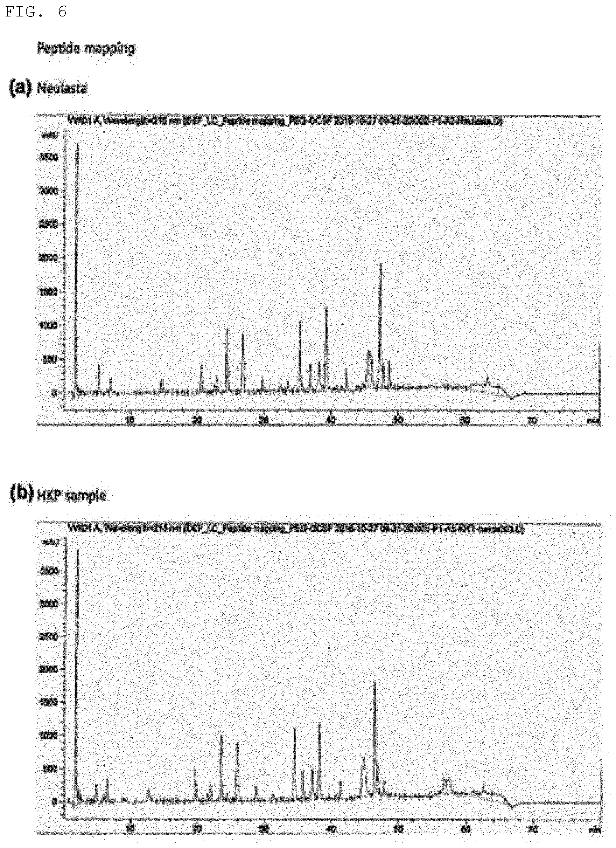

In addition, FIGS. 6 is graphs presenting results of peptide mapping, which is a method of identifying specific protein by comparing a mass spectrometry result of peptides produced by cleaving protein with an enzyme with an existing protein sequence result. Therefore, as presented in FIG. 6, it was confirmed that the HKP sample which is the test group obtained by the preparing method according to the present application and Neulasta which is the control group exhibit a same pattern.

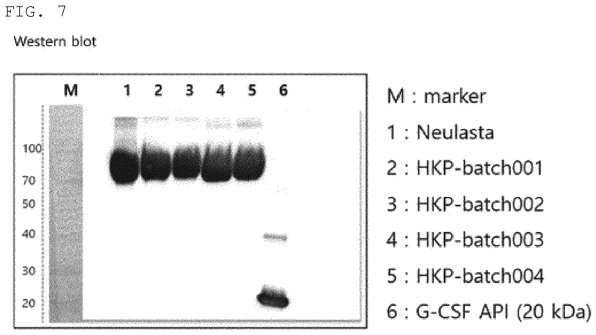

FIG. 7 is a photograph presenting a result of a western blot which is detected by using antibodies to find the presence or absence and an amount of a specific protein. As presented in FIG. 7, as a result of the western blot, protein expression of the HKP sample as the test group obtained by the preparing method according to the present application was confirmed in the same position as that of Neulasta as the control group, and protein expression of G-CSF API was confirmed at the bottom.

From this, the present invention was found that by applying a newly discovered range of pH, the purification by the purification process with only a single step is possible, and as a result, it was also found that an effect of the purification which is equal to or greater than that of Neulasta with multiple steps is exhibited.

Since specific parts of the present invention are specifically described above, it is obvious that for those skilled in the art, such specific technology is only a preferred embodiment, and the scope of the present invention is not limited thereto. Therefore, the substantial scope of the present invention is defined by the appended claims and their equivalents.

### [Industrial Applicability]

The present invention relates to a method for preparing, in a high yield, a conjugate in which a colony stimulating factor and polyol are conjugated, and it was found that an effect of the purification which is equal to or greater than that of Neulasta by a purification process with multiple steps is exhibited only by a purification process with a single step so as to be pragmatically used in development and a preparing process of a therapeutic agent for neutropenia.

## Claims

1. A method for preparing a conjugate in which a colony stimulating factor and polyol are conjugated, the method comprising:
a step of mixing polyol and a colony stimulating factor to produce a mixture;
a step of washing the mixture at pH 3.0 to 7.0 to obtain a washed object; and
a step of eluting the washed object at pH 4.1 to 6.5 to obtain an eluate.

2. The method for preparing a conjugate in which a colony stimulating factor and polyol are conjugated according to claim 1,
wherein the polyol is poly(ethylene glycol) (PEG).

3. The method for preparing a conjugate in which a colony stimulating factor and polyol are conjugated according to claim 1,
wherein the colony stimulating factor is a granulocyte colony stimulating factor (G-CSF).

4. The method for preparing a conjugate in which a colony stimulating factor and polyol are conjugated according to claim 1, further comprising:
a step of mixing the colony stimulating factor in a ratio with mPEG (monomethoxy-polyethylene glycol) which is one of a precursor of the polyol to be 1:3 to prepare the mixture.

5. The method for preparing a conjugate in which a colony stimulating factor and polyol are conjugated according to claim 1, further comprising:
a step of using 20 mM ammonium acetate-acetic acid as a buffer in the washing.

6. The method for preparing a conjugate in which conjugating a colony stimulating factor and polyol are conjugated according to claim 5,
wherein the buffer in the washing is pH 4.8 to 6.8.

7. The method for preparing a conjugate in which a colony stimulating factor and polyol are conjugated according to claim 5,
wherein the buffer in the washing is pH 5.8.

8. The method for preparing a conjugate in which a colony stimulating factor and polyol are conjugated according to claim 1, further comprising:
a step of using 20 mM ammonium acetate-acetic acid as the buffer in the elution.

9. The method for preparing a conjugate in which a colony stimulating factor and polyol are conjugated according to claim 8,
wherein the buffer in the elution is pH 4.5 to 5.5.

10. The method for preparing a conjugate in which a colony stimulating factor and polyol are conjugated according to claim 8,
wherein the buffer in the elution is pH 5.0.
